# EUROPÄISCHE PATENTSCHRIFT

(11) **EP 1 214 280 B1**
(45) Veröffentlichungstag und Bekanntmachung des Hinweises auf die Patenterteilung: **04.02.2004**
(21) Anmeldenummer: 00960526.2
(22) Anmeldetag: 25.08.2000
(51) Int. Cl.: C07C 17/395, C07C 17/383, C07C 19/08

(54) **HERSTELLUNG HOCHREINER FLUORVERBINDUNGEN**
PRODUCTION OF EXTREMELY PURE FLUORINE COMPOUNDS
PRODUCTION DE COMPOSES FLUORES EXTREMEMENT PURS

(30) Priorität: 04.09.1999 DE 19942305
(43) Veröffentlichungstag der Anmeldung: 19.06.2002
(73) Patentinhaber: Solvay Fluor und Derivate GmbH, 30173 Hannover (DE)
(72) Erfinder: BRAUN, Max, 30900 Wedemark (DE); BROSCH, Carsten, 30173 Hannover (DE); GRESS, Heinz, 30457 Hannover (DE)
(74) Vertreter: Fischer, Reiner
(86) Internationale Anmeldenummer: PCT/EP2000/008313
(87) Internationale Veröffentlichungsnummer: WO 2001/017933

(56) Entgegenhaltungen:
- WO-A-97/37955
- GB-A- 2 272 696
- GB-A- 2 318 350
- US-A- 3 218 363

## Beschreibung

Die Erfindung bezieht sich auf ein Verfahren zur Herstellung von gereinigten, teilfluorierten und perfluorierten Kohlenwasserstoffen.

Teilfluorierte Kohlenwasserstoffe können beispielsweise als Treibmittel für die Herstellung von Kunststoffschäumen oder zur Erzeugung von medizinischen Aerosolen verwendet werden.

Perfluorcarbone eignen sich beispielsweise als Blutersatzstoff oder bei der Herstellung von Ultraschallkontrastmitteln.

Perfluorcarbone werden beispielsweise hergestellt, indem man Kohlenwasserstoffe oder teilfluorierte Kohlenwasserstoffe mit elementarem Fluor bzw. fluorabgebenden, höhervalenten Metallfluoriden umgesetzt werden. Für die Herstellung von teilfluorierten Kohlenwasserstoffverbindungen gibt es mannigfaltige Verfahren. Dazu zählen beispielsweise die nicht erschöpfende Fluorierung von Kohlenwasserstoffen mit elementarem Fluor oder fluorabgebenden, höhervalenten Metallfluoriden, der - gewöhnlich katalysierte - Chlor-Fluor-Austausch in flüssiger Phase oder der Gasphase, die Anlagerung von Fluorwasserstoff an ungesättigte Ausgangsverbindungen, oder eine Kombination mehrerer dieser Verfahren. Die erhaltenen,Rohprodukte sind herstellungsbedingt oft durch ungesättigte Kohlenwasserstoffe, durch ungesättigte Chlor und/oder Fluor enthaltende Kohlenwasserstoffe bzw. durch Chlor und gegebenenfalls Fluor substituierte Kohlenwasserstoffe verunreinigt. Die destillative Auftrennung ist oft deswegen schwierig, weil die gewünschten Produkte mit Verunreinigungen azeotrope oder azeotrop ähnliche Gemische bilden. Es ist bereits bekannt, daß man solche Rohprodukte reinigen kann, indem man die ungesättigten Verbindungen bzw. die chlorhaltigen Verunreinigungen mittels Sorbenzien wie Aktivkohle oder Kieselgel oder durch Umsetzung mit Reaktanten mit Wasserstoff, elementarem Fluor oder Chlor reinigen kann.

Das US-Patent 3,218,363 offenbart die Reinigung von Perfluor- und Perfluorchlorkohlenstoffen mit dem Ziel, olefinische oder wasserstoffhaltige Verunreinigungen zu entfernen. Hierzu wird das verunreinigte Rohprodukt mit einem starken Oxidationsmittel behandelt. Dabei kann photochemische Energie eingestrahlt werden. Die WO 97/379 955 offenbart ein Verfahren zur Entfernung olefinischer Verunreinigungen aus 1,1,1,3,3-Pentafluorpropan durch Photochlorierung. Die GB-A 2 318 350 offenbart die Entfernung olefinischer Verunreinigungen aus Hydrochlorofluorethanen durch Photochlorierung.

Aufgabe der vorliegenden Erfindung ist es, ein einfach durchzuführendes, wirksames Verfahren zur Reinigung von Perfluorcarbonen und Fluorkohlenwasserstoffen anzugeben. Diese Aufgabe wird durch das in den Ansprüchen angegebene Verfahren gelöst.

Die Erfindung beruht auf der Beobachtung, daß die Bestrahlung von solchen Rohprodukten mittels Licht einer Wellenlänge von λ > 200 nm die Gewinnung gereinigter Perfluorcarbone bzw. Fluorkohlenwasserstoffe ermöglicht. Ohne daß diese Erklärung einschränkend ausgelegt werden soll, wird als Erklärung angenommen, daß die Perfluorcarbone bzw. Fluorkohlenwasserstoffe durch das eingestrahlte Licht nicht beeinflußt werden, während die Verunreinigungen durch Polymerisation, Chlorabspaltung und Weiterreaktion der gebildeten Radikale und eventueller anderer Reaktionen zu höhersiedenden Produkten reagieren, die sich von den Perfluorcarbonen bzw. Fluorkohlenwasserstoffen leicht abtrennen lassen.

Das erfindungsgemäße Verfahren zur Herstellung von gereinigten gesättigten teilfluorierten und perfluorierten Kohlenwasserstoffen mit 1 bis 10 C-Atomen aus Rohprodukten, die durch Verbindungen verunreinigt sind, die Licht einer Wellenlänge von λ > 200 nm absorbieren, sieht vor, daß man das Rohprodukt mit UV-Strahlung einer Wellenlänge λ > 200 nm bestrahlt und aus dem bestrahlten Reaktionsprodukt gereinigte teilfluorierte oder perfluorierte Kohlenwasserstoffe gewinnt. Man kann aliphatische und cycloaliphatische Kohlenwasserstoffe reinigen.

Vorzugsweise wendet man das Verfahren an, um gereinigte teilfluorierte und perfluorierte Kohlenwasserstoffe herzustellen, die durch Alkene und/oder Alkane verunreinigt sind, welche durch Chlor und gegebenenfalls Fluor substituiert sind.

Teilfluorierte Kohlenwasserstoffe weisen mindestens 1 Fluoratom und mindestens 1 Wasserstoffatom aus.

Man kann auch mit UV-Licht einstrahlen, welches eine Wellenlänge λ unterhalb von 200 nm aufweist, beispielsweise bis herab zu 180 nm. Wichtig ist, daß die Strahlungsquelle im Absorptionsbereich der Verunreinigungen, üblicherweise zwischen 200 bis 250 nm Strahlung abgibt. Dabei muß das abgestrahlte Spektrum nicht kontinuierlich sein, einzelne Wellenlängen im genannten Bereich genügen.

Das erfindungsgemäße Verfahren eignet sich zur Herstellung von gereinigten Verbindungen, insbesondere auch zur Herstellung von hochreinen Verbindungen. Dabei bedeutet der Begriff "gereinigt", daß die erhaltenen Produkte weniger mit Verunreinigungen kontaminiert sind als das Rohprodukt. Der Begriff "hochrein" bedeutet, daß die erhaltenen Produkte von jeder verunreinigung maximal 5 ppm, vorzugsweise maximal 1 ppm aufweisen. Der Grad der Reinigung ist von der Einstrahldauer und der Strahlungsleistung der Lichtquelle abhängig. Je länger und intensiver eingestrahlt wird, desto reineres Produkt kann erhalten werden. Der gewünschte Reinheitsgrad kann durch die üblichen Analysen, insbesondere GC-MS, überprüft werden.

Die Abtrennung der gereinigten Produkte aus dem bestrahlten Gemisch kann durch einfaches Destillieren erfolgen.

Die Temperatur liegt zweckmäßig im Bereich von -30 °C bis +100 °C, denn dort sieden die Verunreinigungen üblicherweise. Bevorzugt arbeitet man bei 0 bis 30 °C. Sie kann gewünschtenfalls aber auch in einem höheren oder tieferen Bereich liegen.

Man bestrahlt vorzugsweise in der Flüssigphase, obwohl auch in der Gasphase bestrahlt werden kann.

Besonders bevorzugt eignet sich das erfindungsgemäße Verfahren zur Herstellung von teil- oder perfluorierten C1-C5-Alkanen (auch Cycloalkanen), insbesondere von gereinigtem bzw. hochreinem Difluormethan, 1,1,1,2-Tetrafluorethan, Pentafluorethan, Pentafluorpropan, vorzugsweise HFC-245fa; Hexafluorpropan, Pentafluorbutan, vorzugsweise HFC-365mfc, Perfluorpropan oder Perfluorbutan.

Die Bestrahlung kann beispielsweise in Quarzgefäßen durchgeführt werden. Gut geeignet ist z. B. Suprasil™, Hersteller Heraeus. Brauchbar sind natürlich auch andere, im benötigten Bereich durchlässige Werkstoffe. Zweckmäßig kühlt man die Lampe mit Preßluft, um die Lichtsorption durch Kühlwasser (das ja teilweise verunreinigt sein kann) zu vermeiden.

Gewünschtenfalls kann man auch in einem 1. Schritt durch Chlorierung (z.B. Photochlorierung) die ungesättigten Verunreinigungen in chlorhaltige Produkte überführen und dann, ohne Zusatz von Reaktanten, die erfindungsgemäße Bestrahlung durchführen.

Mit dem erfindungsgemäßen Verfahren ist auf einfache weise die Reinigung von Fluorkohlenwasserstoffen und Perfluorcarbonen möglich. Bei ausreichend langer Einstrahldauer ist es möglich, hochreine Produkte zu gewinnen, die beispielsweise für die Pharmaanwendung oder in der Elektronikindustrie brauchbar sind.

Ein weiterer Gegenstand der Erfindung ist das bestrahlte Gemisch umfassend teilfluorierte oder perfluorierte Kohlenwasserstoffe mit 1 bis 10 C-Atomen und den bei der Bestrahlung gebildeten Höhersiedern, die bei der Bestrahlung aus chlorierten Alkenen bzw. chlorierten Alkanen gebildet worden sind.

Die folgenden Beispiele sollen die Erfindung weiter erläutern, ohne sie in ihrem Umfang einzuschränken.

### Beispiel 1: Herstellung von gereinigtem 1,1,1,3,3-Pentafluorbutan

Das Rohprodukt enthält als Hauptverunreinigung olefinische C₄ClF₃H₄-Isomere.

### Apparatur:

In einem 1,51 Tauchschacht-Photoreaktor ausgestattet mit einem Quecksilberstrahler TQ 150 von Heraeus Noblelight wurde 365mfc vorgelegt und durch Quarzglas bei Raumtemperatur bestrahlt. Die Lampe wurde mit Preßluft gekühlt, um eine eventuelle Absorption von Licht durch Kühlwasser zu vermeiden.

Das 365mfc enthielt vor Versuchsbeginn folgende Verunreinigungen:
3610 ppm C₄ClF₃H₄ (2 olefinische Isomere)
80 ppm CCl₄
40 ppm ClFC=CCl₂ (1111)
128 ppm Cl₂C=CCl₂ (PCE)
1000 ppm CCl₃F (11)

Weiterhin vorhandene Verunreinigungen ohne Quantifizierung: CHCl₃, C₄H₅F₄Cl, C₄H₄F₃Cl, C₄H₅F₃Cl₂, CH₃CFCl₂.

Nach 180 min Bestrahlung wurde der Versuch abgebrochen. Die durch Photolyse entstandenen langkettigen Verbindungen konnten nun durch Destillation abgetrennt werden und lieferten ein 365mfc mit 840 ppm C₄ClF₃H₄ und 37 ppm, CCl₄. Die anderen Verunreinigungen waren nicht mehr nachweisbar.

### Beispiel 2: Reinigung von 1,1,1,3,3-Pentafluorbutan mit CCl₄ als Hauptverunreinigung

In einem 1,5 l Tauchschacht-Photoreaktor ausgestattet mit einem Quecksilberstrahler,TQ 150 von Heraeus Noblelight wurde 365mfc vorgelegt und durch Quarzglas bei Raumtemperatur bestrahlt. Das 365mfc enthielt vor Versuchsbeginn folgende Verunreinigungen:
20 ppm C₄ClF₃H₄ (2 olefinische Isomere)
80 ppm CCl₄
5 ppm CHCl₃
15 ClF=Cl₂(CFC-1111)
25 ppm CCl₃F (CFC-11)

Weiterhin vorhandene Verunreinigungen ohne Quantifizierung: C₄H₅F₄Cl, C₄H₄F₃Cl, C₄H₅F₃Cl₂, CH₃CFCl₂.

Nach 180 min Bestrahlung wurde der Versuch abgebrochen. Die durch Photolyse entstandenen langkettigen Verbindungen konnten nun durch Destillation abgetrennt werden und lieferten ein 365mfc mit 3 ppm CCl₄ und 1 ppm CHCl₃. Die anderen Verunreinigungen waren nicht mehr nachweisbar.

## Patentansprüche

1. Verfahren zur Herstellung von gesättigten, gereinigten, teilfluorierten und perfluorierten Kohlenwasserstoffen mit 1 bis 10 C-Atomen aus Rohprodukten, die durch Verbindungen verunreinigt sind, die Licht einer Wellenlänge von λ > 200 nm absorbieren, wobei man das Rohprodukt mit UV-Strahlung einer Wellenlänge λ > 200 nm bestrahlt und aus dem bestrahlten Reaktionsprodukt gereinigte teilfluorierte oder perfluorierte Kohlenwasserstoffe gewinnt.

2. Verfahren nach Anspruch 1, **dadurch gekennzeichnet, daß** man bei einer Temperatur von 0 bis 30 °C bestrahlt.

3. Verfahren nach Anspruch 1 oder 2, **dadurch gekennzeichnet, daß** man durch Quarzglas bestrahlt.

4. Verfahren nach einem der vorhergehenden Ansprüche, **dadurch gekennzeichnet, daß** man nach der Bestrahlung eine destillative Trennung des bestrahlten Produktes vornimmt.

5. Verfahren nach Anspruch 1, **dadurch gekennzeichnet, daß** man hochreines 1,1,1,2-Tetrafluorethan, Pentafluorethan, Pentafluorpropan, Hexafluorpropan, Pentafluorbutan, Perfluorpropan oder Perfluorbutan herstellt.

6. Verfahren nach Anspruch 1, **dadurch gekennzeichnet, daß** Alkene, die durch Chlor und/oder Fluor substituiert sein können, als Verunreinigungen enthalten sind.

7. Verfahren nach Anspruch 1, **dadurch gekennzeichnet, daß** durch Chlor und ggf. Fluor substituierte Alkane als Verunreinigung enthalten sind.

8. Verfahren nach Anspruch 1, **dadurch gekennzeichnet, daß** man hochreine gesättigte teilfluorierte oder perfluorierte Kohlenwasserstoffe herstellt.

9. Gemisch aus teilfluorierten oder perfluorierten Kohlenwasserstoffen mit 1 bis 10 C-Atomen und Chlor enthaltenden Höhersiedern, erhalten durch UV-Bestrahlung eines Rohproduktes, welches teilfluorierte oder perfluorierte Kohlenwasserstoffe mit 1 bis 10 C-Atomen und bei λ > 200 nm absorbierende Verunreinigungen, vorzugsweise chlorierte Alkene, chlorierte Alkane, chlorfluorierte Alkene und/oder chlorfluorierte Alkane enthält.

## Claims

1. A process for the preparation of saturated, purified, partially fluorinated and perfluorinated hydrocarbons with 1 to 10 C atoms from crude products which are contaminated by compounds which absorb light of a wavelength of λ > 200 nm, wherein the crude product is irradiated with UV radiation of a wavelength of λ > 200 nm and purified partially fluorinated or perfluorinated hydrocarbons are recovered from the irradiated reaction product.

2. A process according to Claim 1, **characterised in that** irradiation is effected at a temperature from 0 to 30°C.

3. A process according to Claim 1 or 2, **characterised in that** irradiation is effected through quartz glass.

4. A process according to one of the preceding claims, **characterised in that** after irradiation separation of the irradiated product is effected by distillation.

5. A process according to Claim 1, **characterised in that** highly pure 1,1,1,2-tetrafluoroethane, pentafluoroethane, pentafluoropropane, hexafluoropropane, pentafluorobutane, perfluoropropane or perfluorobutane is prepared.

6. A process according to Claim 1, **characterised in that** alkenes which may be substituted by chlorine and/or fluorine are contained as impurities.

7. A process according to Claim 1, **characterised in that** alkanes which may be substituted by chlorine and optionally fluorine are contained as impurities.

8. A process according to Claim 1, **characterised in that** highly pure saturated, partially fluorinated or perfluorinated hydrocarbons are prepared.

9. A mixture of partially fluorinated or perfluorinated hydrocarbons with 1 to 10 C atoms and chlorine-containing higher boilers, obtained by UV irradiation of a crude product which contains partially fluorinated or perfluorinated hydrocarbons with 1 to 10 C atoms and impurities which absorb at λ > 200 nm, preferably chlorinated alkenes, chlorinated alkanes, chlorofluorinated alkenes and/or chlorofluorinated alkanes.

## Revendications

1. Procédé de préparation d'hydrocarbures partiellement fluorés et perfluorés, purifiés, saturés, ayant de 1 à 10 atomes de carbone, à partir de produits bruts contaminés par des composés qui absorbent la lumière ayant une longueur d'onde λ > 200 nm, procédé dans lequel on expose le produit brut à un rayonnement UV ayant une longueur d'onde λ > 200 nm, et, à partir du produit de réaction ainsi irradié, on obtient des hydrocarbures partiellement fluorés ou perfluorés purifiés.

2. Procédé selon la revendication 1, **caractérisé en ce qu'**on irradie à une température de 0 à 30 °C.

3. Procédé selon la revendication 1 ou 2, **caractérisé en ce qu'**on irradie à travers un verre de quartz.

4. Procédé selon l'une des revendications précédentes, **caractérisé en ce qu'**on procède, après l'irradiation, à une séparation par distillation du produit irradié.

5. Procédé selon la revendication 1, **caractérisé en ce qu'**on prépare un 1,1,1,2-tétrafluoroéthane, un pentafluoréthane, un pentafluoropropane, un hexafluoropropane, un pentafluorobutane, un perfluoropropane ou un perfluorobutane de haute pureté.

6. Procédé selon la revendication 1, **caractérisé par** la présence, en tant qu'impuretés, d'alcènes pouvant être substitués par du chlore et/ou du fluor.

7. Procédé selon la revendication 1, **caractérisé par** la présence, en tant qu'impuretés, d'alcanes substitués par du chlore et éventuellement du fluor.

8. Procédé selon la revendication 1, **caractérisé en ce qu'**on prépare des hydrocarbures saturés, partiellement fluorés ou perfluorés, de haute pureté.

9. Mélange d'hydrocarbures partiellement fluorés ou perfluorés ayant de 1 à 10 atomes de carbone et de substances à haut point d'ébullition contenant du chlore, obtenu par exposition à un rayonnement UV d'un produit brut qui contient des hydrocarbures partiellement fluorés ou perfluorés ayant de 1 à 10 atomes de carbone et des composés absorbants à une longueur d'onde λ > 200 nm, de préférence des alcènes chlorés, des alcanes chlorés, des alcènes chlorofluorés et/ou des alcanes chlorofluorés.
